# EUROPÄISCHE PATENTSCHRIFT

(11) **EP 1 362 617 B1**
(45) Veröffentlichungstag und Bekanntmachung des Hinweises auf die Patenterteilung: **21.06.2006**
(21) Anmeldenummer: 03008655.7
(22) Anmeldetag: 15.04.2003
(51) Int. Cl.: A61N 5/10, A61G 13/00, A61B 6/04, A61B 5/055, F16M 7/00

(54) **Verstellbare Patientenlagerungsvorrichtung für ein Strahlentherapiesystem**
Adjustable patient support device for a radiation therapy system
Dispositif réglable de positionnement de patient pour une système de radiotherapie

(30) Priorität: 13.05.2002 DE 10221180
(43) Veröffentlichungstag der Anmeldung: 19.11.2003
(73) Patentinhaber: SIEMENS AKTIENGESELLSCHAFT, 80333 München (DE)
(72) Erfinder: Amann, Karl, Prof., 92274 Gebenbach (DE); Plannerer, Jürgen, 95478 Kemnath (DE); Seufert, Matthias, 91097 Oberreichenbach (DE)

(56) Entgegenhaltungen:
- EP-A- 0 283 083
- WO-A-02/32311
- CH-A- 678 451
- FR-A- 2 757 045
- PATENT ABSTRACTS OF JAPAN vol. 018, no. 198 (C-1187), 7. April 1994 (1994-04-07) & JP 06 000224 A (HITACHI MEDICAL CORP), 11. Januar 1994 (1994-01-11)

## Beschreibung

Die Erfindung betrifft eine Patientenlagerungsvorrichtung für eine Strahlentherapieanlage mit einem Tragelement, das dazu hergerichtet ist, an einer ortsfesten Struktur um eine durch ein Behandlungs-Isozentrum verlaufende, vertikale Iso-Achse drehbar gelagert zu werden, und mit einer an dem Tragelement angebrachten Patientenliege.

DE 38 03 567 A1 offenbart einen nivellierbaren ortsunabhängigen Patientenlagerungstisch. Auch aus FR 2 757 045 A3 ist ein derartiger Patientenlagerungstisch zu entnehmen.

Die Erfindung liegt auf dem Gebiet der Strahlentherapieanlagen, mit denen beispielsweise erkranktes Tumorgewebe mittels harter Röntgenstrahlung, mittels Elektronen oder mittels Gammastrahlen behandelt wird. Aufgrund der hohen Energie der dabei zur Anwendung kommenden Strahlen, die für das erkrankte Gewebe letal ist, besteht die Notwendigkeit, den erkrankten Bereich umgebende Körperzellen zu schonen. Dies geschieht dadurch, dass die Gesamt-Dosisleistung auf mehrere Einstrahlwinkel verteilt wird. Zum einen werden unterschiedliche Einstrahlwinkel dadurch erzeugt, dass der Strahlerkopf während der Bestrahlung um eine horizontale Achse (horizontale Iso-Achse) rotiert, wobei die Bestrahlung entweder bei diskreten Einstrahlwinkeln oder kontinuierlich während der Drehung erfolgt. Außerdem werden unterschiedliche Einfallswinkel dadurch erzeugt, dass die Patientenliege um eine vertikale Achse (vertikale Iso-Achse) rotiert. Die beiden Iso-Achsen schneiden sich in einem sogenannten Behandlungs-Isozentrum.

Zur Bestrahlung eines Patienten wird dieser auf die Patientenliege horizontal aufgelegt, und anschließend lateral und in der Höhe derart positioniert, dass der erkrankte, zu bestrahlende Bereich exakt im Isozentrum zu liegen kommt. Um diese Positionierung exakt vornehmen zu können, wird in der Regel zuvor der Patient in einem Simulator markiert und/oder der Tumor zuvor mittels eines bildgebenden Gerätes exakt lokalisiert. Um eine ausreichende Positionierung des erkrankten Gewebes im Isozentrum zu erreichen, werden dabei sogar die beispielsweise in einem Computertomographen gewonnenen 3D-Daten auf die Strahlentherapieanlage übertragen und dort zur Patientenpositionierung verwendet bzw. es wird der Patient mittels an ihm angebrachter Markierungen auf der Therapieanlage - zum Beispiel unter Verwendung eines "Laserkreuzes" - ausgerichtet.

Da die Gantry mit dem Strahlerkopf um eine horizontale Achse um 360° schwenken kann, muss die Patientenliege oder Tischplatte im Bereich des Isozentrums auch von unten voll zugänglich sein. Daher wird die Patientenliege in der Regel von einer außermittig zum Isozentrum angeordneten Hubsäule getragen. Unter anderem aus Gründen der Bauhöhe wird ein die Patientenliege tragendes, um die vertikale Iso-Achse drehbar gelagertes Tragelement in der Regel zumindest teilweise in einer Bodensenke angebracht. Strahlentherapieanlagen oder Patientenlagerungsvorrichtungen der genannten Art sind beispielsweise beschrieben in EP 0 283 083 A1, in der deutschen Offenlegungsschrift 20 46 207 sowie in WO 88/01848.

Aus JP 06000224 A ist eine Strahlentherapieanlage zu entnehmen, bei der auf elektronischem Weg die Höheneinstellung der Patientenliege im Hinblick auf eine Abweichung vom Isozentrum überprüft und bei Bedarf korrigiert wird.

Der Erfindung liegt die Aufgabe zugrunde, die Präzision bei der Bestrahlung in einer Strahlentherapieanlage gegenüber bekannten Anlagen zu erhöhen, um somit das erkrankte Gewebe zielgerichteter behandeln zu können und letztlich auch eine Strahlenbelastung zu erreichen.

Diese Aufgabe wird gemäß der Erfindung bezogen auf die eingangs genannte Patientenlagerungsvorrichtung gelöst durch mindestens ein stufenlos einstellbares, der Ausrichtung des Tragelements dienenden Nivelliermittel das zur Ausrichtung des Trageelements in der Horizontalen dient.

Die Erfindung geht von der Erkenntnis aus, dass es für eine Strahlentherapieanlage insbesondere auf eine sehr hohe Präzision der Nivellierung des Tragelements ankommt. Insbesondere muss das Tragelement in der Horizontalen nivelliert werden. Damit wird vor allem eine exakte Patientenpositionierung unabhängig von der Winkelstellung der isozentrischen Rotation sowie außerdem eine Reduzierung der Bewegungskräfte bei manueller Betätigung der Rotation erreicht, indem eine Schrägstellung der Drehachse bis auf ein Mindestmaß vermeidbar ist. Die Erfindung geht weiterhin von der Erkenntnis aus, dass mit modernen Strahlentherapieanlagen ein Präzisionsmaß erreicht ist, für welches eine herkömmliche, starre Verankerung des Tragelements in der ortsfesten Struktur nicht mehr ausreicht. Bislang war es allenfalls üblich, eine nur grobe Nivellierung durch metallische Unterlegplatten, sogenannte Shims, vorzunehmen. Die Erfindung geht von dieser bislang üblichen, starren Verankerung ab und schlägt als neuen Weg die stufenlose Nivellierung ein.

Neben dem bereits genannten Präzisionsvorteil wird mit der Erfindung außerdem der Vorteil erreicht, dass die Installation gegenüber der erwähnten Vorgehensweise mit metallischen Unterlegplatten vereinfacht und erheblich beschleunigt ist.

Nach einer bevorzugten Ausgestaltung ist das Tragelement dazu hergerichtet, direkt oder indirekt über das Nivelliermittel an der ortsfesten Struktur angebracht zu werden, vorzugsweise für den Dauerbetrieb. Durch eine Nivellierung direkt im Bereich der Auflagefläche wird eine besonders einfache Konstruktion erreicht.

Außerdem bevorzugt ist auch nach erfolgter Installation des Tragelements an der ortsfesten Struktur das Nivelliermittel noch einstellbar. Damit wird ein einfaches Nachjustieren ohne Deinstallation der sehr schweren Patientenlagerungsvorrichtung möglich. Die Erfindung geht davon aus, dass bei sehr präzise arbeitenden, modernen Strahlentherapieanlagen ein solches Nachjustieren im Laufe der Zeit nötig werden kann, um die hohe Präzision zu halten.

Nach einer besonders bevorzugten Ausgestaltung ist das Nivelliermittel als Nivellierschuh ausgebildet. Ein solcher Nivellierschuh besteht beispielsweise aus einer Grundplatte, einer Deckplatte und einem dazwischen angeordneten verstellbaren Keil.

Entsprechende, hierfür in Frage kommende Nivellierschuhe sind beispielsweise beschrieben in DE 44 29 813 C2, DE 44 00 164 A1, DE 32 28 606 A1 oder CH 678 451 A5. Der Nivellierschuh gemäß DE 44 29 813 C2 umfasst eine Grundplatte, eine Deckplatte und einen dazwischen angeordneten Keil, der mittels einer in ihm drehbar und axial unverschieblich gelagerten, horizontalen Schraubspindel, auf die ein in die Grundplatte und die Deckplatte eingreifendes, gegenüber diesen in senkrechter Richtung frei verschiebliches und in Richtung der Schraubspindel in diesen festgelegtes Verankerungsglied aufgeschraubt ist, mehr oder weniger tief zwischen Grundplatte und Deckplatte einschiebbar ist, so dass der senkrechte Abstand von Grundplatte und Deckplatte dadurch veränderbar ist.

Bevorzugt ist das Nivelliermittel bei der Patientenlagerungsvorrichtung derart angeordnet, dass eine horizontale Bewegung des Keils eine Vertikalbewegung des Tragelements bewirkt.

Dabei weist das Nivelliermittel vorzugsweise eine horizontal gelagerte Gewindespindel zum Verstellen des Keils auf, die insbesondere maßgeblich in Richtung zur vertikalen Iso-Achse zeigt.

Nach einer besonders bevorzugten Ausgestaltung weist die Patientenlagerungsvorrichtung mindestens drei, insbesondere genau drei, Nivelliermittel auf. Die Erfindung geht nämlich von der Erkenntnis aus, dass - entgegen einer bislang üblichen Vorgehensweise, wonach zur Nivellierung an 16 Stellen Befestigungsbohrungen und gegebenenfalls untergelegte Metallplatten vorgesehen wurden - trotz des hohen Gewichts einer Patientenlagerungsvorrichtung für eine Strahlentherapieanlage eine Dreipunktlagerung möglich ist. Dem liegt insbesondere auch die Erkenntnis zugrunde, dass die bereits erwähnten Nivellierschuhe hinsichtlich ihrer Belastbarkeit für eine derartige Dreipunktlagerung ausreichend sind. Durch die Befestigung der Bauteile für die vertikale isozentrische Rotation an nur noch drei Punkten kann in vorteilhafter Weise auf eine stabile Bodenwanne aus Metall mit einem großflächig bearbeiteten Befestigungsflansch verzichtet werden. Im einfachsten Fall müssen nur noch drei Schrauben justiert oder gelöst werden.

Das Tragelement kann zumindest teilweise in einer als ortsfeste Struktur fungierenden Bodensenke angebracht sein. Als ortsfeste Strukturen kommen aber auch eine Standsäule für eine Gantry oder die Gantry selbst in Frage.

Insbesondere im Zusammenhang mit der bereits erwähnten Dreipunktlagerung ist es zweckmäßig, dass die Bodensenke an ihrer Grundfläche eine Montagefläche aus Beton aufweist, in der das Nivelliermittel unmittelbar verankert ist. Die Verankerung kann beispielsweise mit Spezialdübeln im Betonboden erfolgen, wobei insbesondere pro Nivellierschuh ein Dübel ausreichend wäre.

Das Tragelement kann außerdem mit der Patientenliege über eine, bevorzugt außerhalb der vertikalen Iso-Achse angebrachte, Hubsäule in Verbindung stehen.

Um eine Bewegbarkeit des Strahlerkopfs um 360° um die horizontale Iso-Achse zu gewährleisten, ist es zweckmäßig, dass das Tragelement einen Ausleger zur Aufnahme der Hubsäule aufweist.

Die Patientenliege kann insbesondere derart positioniert oder positionierbar sein, dass sie an einer Stelle von der vertikalen Iso-Achse durchdrungen ist.

Im Rahmen der Erfindung liegt auch noch eine Strahlentherapieanlage mit einer Patientenlagerungsvorrichtung nach der Erfindung. Wegen diesbezüglicher Vorteile und bevorzugter Ausführungen sei auf die Patientenlagerungsvorrichtung verwiesen.

Ausführungsbeispiele einer Patientenlagerungsvorrichtung und einer Strahlentherapieanlage nach der Erfindung werden nachfolgend anhand der Figuren 1 bis 4 näher erläutert. Es zeigen:
- Figur 1: eine schematische Gesamtansicht einer Strahlentherapieanlage nach der Erfindung,
- Figur 2: eine perspektivische Detailsicht der Patientenlagerungsvorrichtung der Strahlentherapieanlage der Figur 1,
- Figur 3: eine Querschnittsdarstellung der Patientenlagerungsvorrichtung der Figur 2 und
- Figur 4: eine schematische Darstellung eines zur Nivellierung der Patientenlagerungsvorrichtung der Figuren 2 und 3 verwendetes Nivelliermittel.

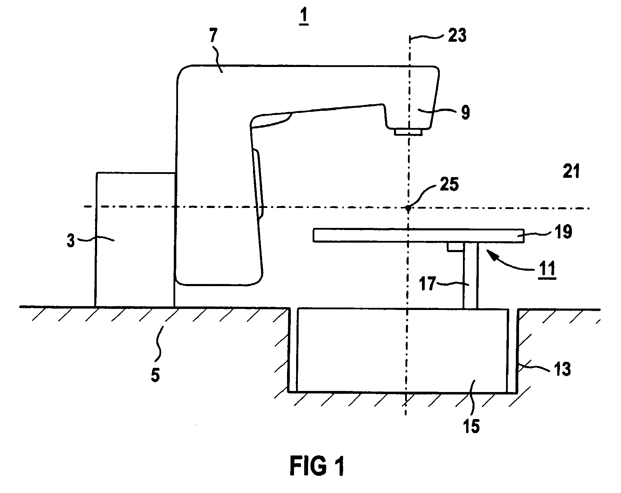

**Figur 1** zeigt eine insgesamt mit dem Bezugszeichen 1 bezeichnete Strahlentherapieanlage. Die Strahlentherapieanlage 1 umfasst eine Bodensäule 3, die auf einem Boden 5 eines Untersuchungsraums oder eines Gebäudes befestigt ist. An der Bodensäule 3 ist eine Gantry 7 oder ein Gestell drehbar gelagert, welches an einem Ende eine Bestrahlungsquelle 9, beispielsweise eine Röntgenquelle oder eine Gammastrahlenquelle, aufweist.

Die Strahlentherapieanlage 1 umfasst außerdem eine Patientenlagerungsvorrichtung 11. Diese ist mittels eines Tragelements 15 in einer Bodensenke 13 rotierbar gelagert. An dem Tragelement 15 ist über eine Hubsäule 17 eine Patientenliege 19 oder Tischplatte zur Auflage eines nicht explizit dargestellten Patienten angebracht.

Die Gantry 7 mit der Bestrahlungsquelle 9 ist um eine horizontale Iso-Achse 21 rotierbar, die Patientenlagerungsvorrichtung 11, insbesondere die Patientenliege 19, um eine vertikale Iso-Achse 23. Die Rotation um die vertikale Iso-Achse 23 erlaubt, dass mehr unterschiedliche Einstrahlwinkel im Raum erreicht werden, als dies nur bei Rotation der Gantry um die horizontale Iso-Achse 21 möglich wäre.

Die beiden Iso-Achsen 21, 23 sind derart orientiert, dass sie sich in einem gemeinsamen Behandlungs-Isozentrum 25 schneiden.

In dieses Behandlungs-Isozentrum 25 wird der auf die Patientenliege 19 aufgelegte Patient, beispielsweise mit einem zu behandelnden Tumor, positioniert.

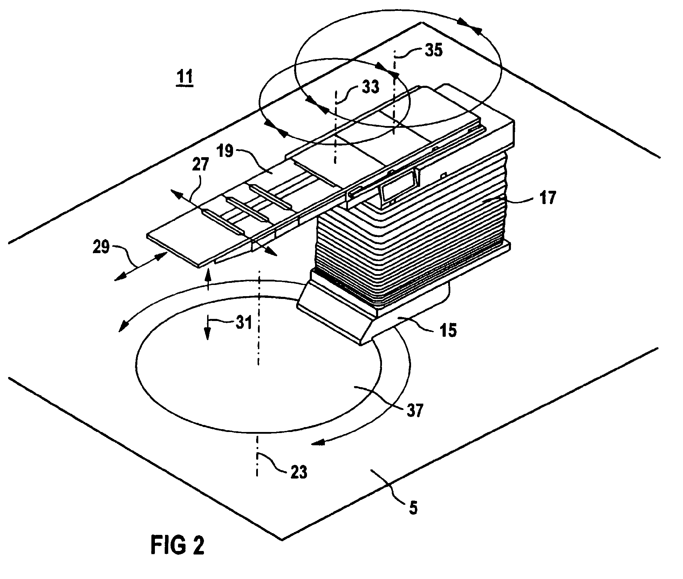

Wie in **Figur 2** im Einzelnen dargestellt ist, sind zu diesem Zwecke translatorische Bewegungen der Patientenliege 19 in einer Lateralrichtung 27 und in einer Längsrichtung 29, beide in der horizontalen Ebene, möglich. Außerdem ist eine Hubhöhenverstellung mit der Hubsäule 17 in Höhenrichtung 31 möglich. Die Hubsäule garantiert einen großen Verstellbereich, um den Patienten z.B. auch weit weg vom Strahlerkopf zu positionieren, damit eine maximale Feldgröße genutzt werden kann, da der Strahl divergiert.

Da die Gantry 7 um die horizontale Iso-Achse 21 um 360° schwenkbar ist, ist die Tischplatte 19 im Bereich des Isozentrums 25 auch von unten voll zugänglich, weshalb die Hubsäule 17 außermittig angeordnet ist.

In Figur 2 sind weiterhin noch zwei Rotationsachsen zu erkennen: Um eine erste vertikale Achse 33 ist die Patientenliege 19 auf der Hubsäule 17 um ± 180° schwenkbar. Um eine zweite vertikale Achse 35 ist die Hubsäule 17 ihrerseits um ± 180° schwenkbar.

Die Rotationsmöglichkeiten um die vertikalen Achsen 33, 35 erlauben eine Parallelversetzung der Patientenliege 19, um einen Patienten bei Bedarf in einem Krankenbett zu behandeln. Außerdem kann durch eine kombinierte Bewegung um beiden Achsen der laterale Verstellbereich der Tischplatte 19 erweitert werden.

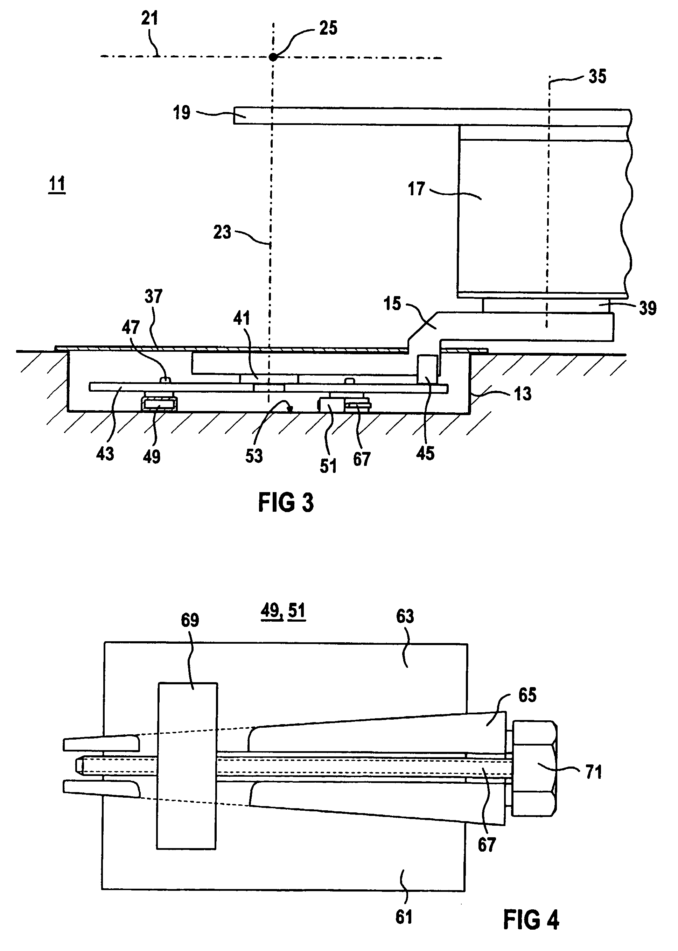

In Figur 2 ist außerdem ersichtlich, dass das Tragelement 15 mit einem Auslegerteil aus der ansonsten von einer mitrotierbaren Abdeckplatte 37 abgedeckten Bodensenke 13 herausragt. Die Abdeckplatte 37 ist abnehmbar, um an die darunter liegenden Konstruktionselemente zu gelangen, die in Figur 3 näher dargestellt sind.

In **Figur 3** ist im Detail ersichtlich, dass die Hubsäule 17 über ein Säulenrotationslager 39 auf dem über die Bodensenke 13 oder Bodenwanne hinausragenden, auslegerartigen Teil des Tragelements 15 gelagert ist. Das armartige Tragelement 15 ist seinerseits über ein Basislager 41 an einer Basisplatte 43 drehbar um die vertikale Iso-Achse 23 gelagert. Die Rotation um diese Achse wird durch eine motorgetriebene, das Tragelement 15 unterstützende Stützrolle 45 gewährleistet, die auf einer Kreisbahn auf der Basisplatte 43 rollbar ist.

Die Basisplatte 43 ist über Befestigungsschrauben 47 mit insgesamt drei, in äquidistantem azimutalem Abstand um die vertikale Iso-Achse 23 angeordnete Nivelliermittel 49, 51 verbunden, die ihrerseits auf einer aus Beton bestehenden Montagefläche 53 der Bodensenke 13 montiert sind. Durch die drei Auflagepunkte ist die Lagerung statisch bestimmt. Die Dreipunktlagerung erlaubt eine zeit- und kostengünstige Montage. Die als Nivellier- oder Maschinenschuhe ausgebildeten Nivelliermittel 49, 51 sind über Spezialdübel unmittelbar im Betonboden verankert. Eine metallische Auskleidung der Bodenwanne 13 ist nicht erforderlich.

**Figur 4** zeigt beispielhaft und schematisch ein in Frage kommendes Nivelliermittel 49, 51. Dieses weist eine Grundplatte 61, eine Deckplatte 63 und einen dazwischen angeordneten Keil 65 auf, der mittels einer in ihm drehbar und axial unverschieblich gelagerten, horizontalen Schraub- oder Gewindespindel 67 mehr oder weniger tief zwischen Grundplatte 61 und Deckplatte 63 einschiebbar ist, so dass der senkrechte Abstand von der Grundplatte 61 zur Deckplatte 63 dadurch veränderbar ist. Auf die Schraubspindel 67 ist ein in die Grundplatte 61 und in die Deckplatte 63 eingreifendes, gegenüber diesen in senkrechter Richtung frei verschiebliches und in Richtung der Schraubspindel 67 in diesen festgelegtes Verankerungsglied 69 aufgeschraubt. Die Gewindespindel 67 ist von außen über einen Schraubkopf 71 manuell antreibbar.

Die Nivelliermittel 49, 51 (siehe Figur 3) sind derart angeordnet, dass durch eine horizontale Bewegung des Keils 65 eine Vertikalbewegung des Tragelementes 15 bewirkt ist. Insbesondere ist die Gewindespindel 67 horizontal gelagert und radial bezüglich der vertikalen Iso-Achse 23 angeordnet, so dass der Schraubkopf 71 in einfacher Weise von außen durch Servicepersonal zugänglich ist, nachdem die Abdeckplatte 37 abgenommen wurde.

Durch die Verwendung der Nivellierelemente 49, 51 kann die isozentrische Rotation der Therapieliege jederzeit ohne Neuinstallation nivelliert werden. Bei Verwendung einer Feingewindespindel ist ein sehr geringes Verstelldrehmoment und somit eine sehr präzise Nivellierung erreichbar. Es ist nur ein kompaktes Werkzeug nötig, welches vom Bedienpersonal in einfacher Weise in die Bodensenke 13 eingebracht werden kann, ohne beim Hantieren behindert zu sein.

Zur Nachrüstung bestehender Bodenwannen mit metallischen Auskleidungen können Adapterbleche eingebracht werden, die insbesondere zwischen den Nivellierelementen 49, 51 und dem metallischen Bereich der Bodenwanne zwischengelegt werden.

## Patentansprüche

1. Patientenlagerungsvorrichtung (11) für eine Strahlentherapieanlage (1),
mit einem Tragelement (15), das dazu hergerichtet ist, an einer ortsfesten Struktur um eine durch ein Behandlungs-Isozentrum (25) verlaufende, vertikale Iso-Achse (23) drehbar gelagert zu werden,
und mit einer an dem Tragelement (15) angebrachten Patientenliege (19),
**gekennzeichnet** d u r c h
mindestens ein stufenlos einstellbares Nivelliermittel (49, 51), das zur Ausrichtung des Tragelements (15) in der Horizontalen dient.

2. Patientenlagerungsvorrichtung (11) nach Anspruch 1
**dadurch gekennzeichnet, dass**
das Tragelement (15) dazu hergerichtet ist, direkt oder indirekt über das Nivelliermittel (49, 51) an der ortsfesten Struktur angebracht zu werden.

3. Patientenlagerungsvorrichtung (11) nach Anspruch 1 oder 2,
**dadurch gekennzeichnet, dass**
auch nach erfolgter Installation des Tragelements (15) an der ortsfesten Struktur das Nivelliermittel (49, 51) einstellbar ist.

4. Patientenlagerungsvorrichtung (11) nach einem der Ansprüche 1 bis 3,
**dadurch gekennzeichnet, dass**
das Nivelliermittel (49, 51) als Nivellierschuh ausgebildet ist.

5. Patientenlagerungsvorrichtung (11) nach einem der Ansprüche 1 bis 4,
**dadurch gekennzeichnet, dass**
das Nivelliermittel (49, 51) eine Grundplatte (61), eine Deckplatte (63) und einen dazwischen angeordneten verstellbaren Keil (65) umfasst.

6. Patientenlagerungsvorrichtung (11) nach Anspruch 5,
**dadurch gekennzeichnet, dass**
das Nivelliermittel (49, 51) derart angeordnet ist, dass eine horizontale Bewegung des Keils (65) eine Vertikalbewegung des Tragelements (15) bewirkt.

7. Patientenlagerungsvorrichtung (11) nach Anspruch 5 oder 6,
**dadurch gekennzeichnet, dass**
das Nivelliermittel (49, 51) eine horizontal gelagerte Gewindespindel (67) zum Verstellen des Keils (65) aufweist.

8. Patientenlagerungsvorrichtung (11) nach Anspruch 7,
**dadurch gekennzeichnet, dass**
die Gewindespindel (67) maßgeblich in Richtung zur vertikalen Iso-Achse (23) zeigt.

9. Patientenlagerungsvorrichtung (11) nach einem der Ansprüche 1 bis 8,
**dadurch gekennzeichnet, dass**
mindestens drei, insbesondere genau drei, Nivelliermittel (49, 51) vorhanden sind.

10. Patientenlagerungsvorrichtung (11) nach einem der Ansprüche 1 bis 9,
**dadurch gekennzeichnet, dass**
das Tragelement (15) zumindest teilweise in einer als ortsfeste Struktur fungierenden Bodensenke (13) angebracht ist.

11. Patientenlagerungsvorrichtung (11) nach einem der Ansprüche 1 bis 10,
**dadurch gekennzeichnet, dass**
die Bodensenke (13) an ihrer Grundfläche eine Montagefläche (53) aus Beton aufweist, in der das Nivelliermittel (49, 51) unmittelbar verankert ist.

12. Patientenlagerungsvorrichtung (11) nach einem der Ansprüche 1 bis 11,
**dadurch gekennzeichnet, dass**
das Tragelement (15) mit der Patientenliege (19) über eine, bevorzugt außerhalb der vertikalen Iso-Achse (23) angebrachte, Hubsäule (17) in Verbindung steht.

13. Patientenlagerungsvorrichtung (11) nach einem der Ansprüche 1 bis 12,
**dadurch gekennzeichnet, dass**
das Tragelement (15) einen Ausleger zur Aufnahme der Hubsäule (17) aufweist.

14. Patientenlagerungsvorrichtung (11) nach einem der Ansprüche 1 bis 13,
**dadurch gekennzeichnet, dass**
die Patientenliege (19) derart positioniert oder positionierbar ist, dass sie an einer Stelle von der vertikalen Iso-Achse (23) durchdrungen ist.

15. Strahlentherapieanlage (1) mit einer Patientenlagerungsvorrichtung (11) nach einem der vorangehenden Ansprüche 1 bis 14.

## Claims

1. Patient support device (11) for a radiation therapy system (1) comprising,
a load-bearing element (15) supported rotatably on a stationary structure about a vertical iso-axis extending through a treatment iso-centre, and with a patient support (19) mounted on the load-bearing element (15), **characterised by**
at least one continuously variable levelling device (49,51) serving to orient the load-bearing element (15) horizontally.

2. Patient support device (11) according to claim 1
**characterised in that**
the load-bearing element (15) is mounted on the stationary structure directly or indirectly via the levelling device (49, 51).

3. Patient support device (11) according to claim 1 or 2,
**characterised in that**
the levelling device (49,51) remains adjustable after the installation of the load-bearing element (15) on the stationary structure.

4. Patient support device (11) according to claims 1 to 3,
**characterised in that**
the levelling device (49,51) comprises a levelling shoe.

5. Patient support device (11) according to one of claims 1 to 4,
**characterised in that**
the levelling device (49, 51) includes a bottom plate (61), a top plate (63), and an adjustable wedge (65) disposed between the bottom and top plates.

6. Patient support device (11) according to claim 5,
**characterised in that**
the levelling device (49, 51) is disposed such that a horizontal motion of the adjustable wedge (65) causes a vertical motion of the load-bearing element (15).

7. Patient support device (11) according to claim 5 or 6
**characterised in that**
the levelling device (49, 51) has a horizontally supported threaded spindle (67) for adjusting the wedge (65).

8. Patient support device (11) according to claim 7,
**characterised in that**
the spindle (67) points substantially in the direction of the vertical iso-axis (23).

9. Patient support device (11) according to one of claims 1 to 8,
**characterised in that**
at least three, in particular precisely three, levelling devices (49, 51) are present.

10. Patient support device (11) according to one of claims 1 to 9,
**characterised in that**
the load-bearing element (15) is mounted at least in part in a floor well (13) functioning as the stationary structure.

11. Patient support device (11) according to one of claims 1 to 10,
**characterised in that**
the floor (13) on its bottom face, has a mounting face (53) of concrete, in which the levelling device is anchored directly.

12. Patient support device (11) according to one of claims 1 to 11,
**characterised in that**
the load-bearing element (15) is connected to the patient support (19) via a lifting column (17) mounted preferably outside the vertical iso-axis (23).

13. Patient support device (11) according to one of claims 1 to 12, **characterised in that**
the load-bearing element (15) has a cantilevered arm for receiving the lifting column (17).

14. Patient support device (11) according to one of claims 1 to 13,
**characterised in that**
the patient support (19) is positioned or can be positioned such that it is penetrated by the vertical iso-axis (23) at one point.

15. Radiation therapy system (1) with a patient support device (11) according to one of the preceding claims 1 to 14.

## Revendications

1. Dispositif (11) de support de patient pour une installation (1) de radiothérapie,
comportant un élément (15) porteur qui est conçu pour être monté à rotation, autour d'un axe (23) isométrique vertical passant par un isocentre (25) de traitement, sur une structure à poste fixe,
et comportant une table de patient (19) installée sur l'élément porteur (14),
**caractérisé par** au moins un moyen (49, 51) de mise à niveau réglable en continu, qui sert à orienter l'élément (15) porteur à l'horizontal.

2. Dispositif (11) de support de patient suivant la revendication 1, **caractérisé en ce que** l'élément (15) porteur est conçu pour être installé sur la structure à poste fixe directement ou indirectement, par le moyen (49, 51) de mise à niveau.

3. Dispositif (11) de support de patient suivant la revendication 1 ou 2, **caractérisé en ce que** le moyen (49, 51) de mise à niveau est réglable même une fois effectuée l'installation de l'élément (15) porteur sur la structure à poste fixe.

4. Dispositif (11) de support de patient suivant l'une des revendications 1 à 3, **caractérisé en ce que** le moyen (49, 51) de mise à niveau est réalisé sous forme de sabot de mise à niveau.

5. Dispositif (11) de support de patient suivant l'une des revendications 1 à 4, **caractérisé en ce que** le moyen (49, 51) de mise à niveau comprend une plaque de base (61), une plaque de recouvrement (63) et une cale (65) réglable disposée entre ces plaques.

6. Dispositif (11) de support de patient suivant la revendication 5, **caractérisé en ce que** le moyen (49, 51) de mise à niveau est disposé de telle sorte qu'un mouvement horizontal de la cale (65) produit un mouvement vertical de l'élément (15) porteur.

7. Dispositif (11) de support de patient suivant la revendication 5 ou 6, **caractérisé en ce que** le moyen (49, 51) de mise à niveau comporte pour régler la cale (65) une broche (67) filetée montée horizontalement.

8. Dispositif (11) de support de patient suivant la revendication 7, **caractérisé en ce que** la broche (67) filetée est pointée essentiellement en direction de l'axe (23) isométrique vertical.

9. Dispositif (11) de support de patient suivant l'une des revendications 1 à 8, **caractérisé en ce qu'**il est prévu au moins trois, et notamment précisément trois, moyens (49, 51) de mise à niveau.

10. Dispositif (11) de support de patient suivant l'une des revendications 1 à 9, **caractérisé en ce que** l'élément (15) porteur est installé au moins partiellement dans une fosse (13) servant de structure à poste fixe.

11. Dispositif (11) de support de patient suivant l'une des revendications 1 à 10, **caractérisé en ce que** la fosse (13) comporte sur sa surface de fond une surface (53) de montage en béton, dans laquelle est directement ancré le moyen (49, 51) de mise à niveau.

12. Dispositif (11) de support de patient suivant l'une des revendications 1 à 11, **caractérisé en ce que** l'élément (15) porteur est relié à la table de patient (19) par l'intermédiaire d'une colonne (17) de levage installée de préférence à l'extérieur de l'axe (23) isométrique vertical.

13. Dispositif (11) de support de patient suivant l'une des revendications 1 à 12, **caractérisé en ce que** l'élément (15) porteur comporte un bras en porte-à-faux pour recevoir la colonne (17) de levage.

14. Dispositif (11) de support de patient suivant l'une des revendications 1 à 13, **caractérisé en ce que** la table de patient (19) est ou peut être positionnée de telle sorte qu'elle est traversée en un point par l'axe (23) isométrique vertical.

15. Installation (1) de radiothérapie pourvue d'un dispositif (11) de support de patient suivant l'une des revendications précédentes 1 à 14.
